# EUROPÄISCHE PATENTSCHRIFT

(11) **EP 2 853 246 B1**
(45) Veröffentlichungstag und Bekanntmachung des Hinweises auf die Patenterteilung: **24.04.2019**
(21) Anmeldenummer: 14003012.3
(22) Anmeldetag: 01.09.2014
(51) Int. Cl.: A61F 5/44

(54) **Sichtschutz fuer Hygienebeutel**
Visibility protection for hygiene bag
Protection de la visibilité pour sachet hygiénique

(30) Priorität: 25.09.2013 DE 102013015966
(43) Veröffentlichungstag der Anmeldung: 01.04.2015
(73) Patentinhaber: Human Nutrition GmbH, 55234 Bechtolsheim (DE)
(72) Erfinder: Wendel, Hermann J., 55288 Armsheim (DE)
(74) Vertreter: Zellentin & Partner mbB Patentanwälte

(56) Entgegenhaltungen:
- WO-A1-02/094143
- DE-U1-202005 016 479
- DE-U1-202013 002 466
- US-A- 5 759 180

## Beschreibung

Die vorliegende Erfindung betrifft einen Hygienebeutel mit Sichtschutz. Hygienebeutel zum Sammeln von Körperflüssigkeiten, insbesondere Urinbeutel, sind in der Pflege bekannt und werden täglich millionenfach verwendet. Urinbeutel dienen dazu, eine Körperflüssigkeit wie Urin, der mittels Katheter aus der Blase abgeleitet wird, aufzufangen und sicher zu verwahren. Ist ein vorgegebener Füllstand erreicht, erfolgt eine Entleerung, die möglicht sicher und vollständig gewährleistet sein soll. Urinbeutel können auch als Ersatz der Blase bezeichnet werden. Sie werden sowohl bei Männern als auch bei Frauen angewendet. Die Verbindung zur Blase und zum Blaseninneren wird mittels eines Katheters hergestellt. Üblicherweise wird der Katheter in die Urethra geschoben und mit Hilfe eines wassergefüllten Ballons in der Blase festgelegt. Der abfließende Urin wird in dem Beutel gesammelt.

Ein solcher Urinbeutel umfasst in der Regel einen Flüssigkeitssammelbehälter (kurz Behälter), der typischerweise aus zwei miteinander verschweißten Folien besteht und einen Anschlussschlauch, der mit dem Katheter verbunden wird. Das Volumen des Behälters beträgt normalerweise 0,5 bis 4 L. Häufig ist auch ein Abfluss zum Entleeren vorgesehen, zumeist ein Schlauch mit einer Ritsch-Ratsch-Klemme. Unzählige Varianten und Ausführungsformen von Urinbeuteln bzw. Hygienebeuteln sind möglich und in Gebrauch. Diese sind dem Fachmann bekannt und bedürfen hier keiner näheren Beschreibung. Beispielhaft sei auf DE 202005001925 T2, EP 847742 B1 und EP 748620 B1 verwiesen.

Eine häufige Gestaltung sieht vor, dass die Vorderseite des Beutels mit einer Skala versehen ist, um die gesammelte Urinmenge abschätzen zu können. Außerdem sind die Beutel, zumindest die Vorderwand, in der Regel durchsichtig, damit Ärzte und Pflegepersonal die Qualität des Urins beurteilen können.

Patienten empfinden die durchsichtigen Beutel jedoch häufig als unangenehm. Man sieht oft in Krankenhäusern, dass Betroffene, die ihre Urinbeutel mittragen bzw. am Rollstuhl befestigen, den Urinbeutel in einer Tragetasche verstauen. Dies ist nicht einem leichteren Tragen geschuldet, sondern mehr der Tatsache, dass Betroffene sich durch den transparenten Beutel in der Intimsphäre verletzt fühlen, den Urin nicht öffentlich ausstellen möchten.

Es werden bereits Möglichkeiten angeboten, wie die Intimsphäre besser geschützt werden kann. So sind Taschen bekannt, die aus Baumwolle gefertigt sind und um den Beutel gelegt werden können. Diese haben eine Öffnung an der Oberseite, aus der der Schlauch, der die Verbindung mit dem Katheter darstellt, geführt werden kann und manchmal eine Öffnung an der Unterseite zum Entleeren des Beutels. Auch Taschen aus anderen Materialien sind bekannt und werden für denselben Zweck genutzt. Beispielhaft für diese Vorschläge sind DE 20 2013 002 466 U1, DE 20 2013 000 927 U1, DE 20 2012 001 065 U1, DE 20 2005 016 479 U1, DE 202 07 270 U1 und US 5,135,519 A.

Alle diese Taschen haben den großen Nachteil, dass die Qualität des Urins nicht beurteilt werden kann. Auch die Füllmenge kann nicht abgelesen werden, da die Taschen die auf den Beutel aufgedruckte Skala verdecken. Für das Entleeren sind viele der Taschen umständlich, da der Beutel zum Entleeren häufig erst aus der Tasche ausgepackt werden muss, danach entleert und dann wieder eingepackt werden muss. Das kann dazu führen, dass die Tasche durch den Urin verschmutzt wird. Außerdem sind solche Taschen vergleichsweise teuer.

Auch in Bezug auf Stomabeutel sind bereits Sichtschutzeinrichtungen vorgeschlagen worden. Die US 4,533,355 A schlägt eine Hose vor, die den Stomabeutel in einer Tasche oder gedoppelten Vorderpartie aufnimmt und zusätzlich eine oberhalb der Aufnahme befestigte Abdeckung vorsieht. Gemäß US 5,759,180 A, die als nächstliegender Stand der Technik angesehen wird, soll eine Abdeckung auf die Vorderseite des Stomabeutels aufgeklebt werden. Auch diese Vorschläge erlauben bei Übertragung auf Urinbeutel nicht die gewünschte einfache Kontrolle von Füllstand und Urinqualität. Es besteht daher weiter die Aufgabe einen Sichtschutz für Hygienebeutel, besonders Urinbeutel, bereitzustellen, der die Intimsphäre schützt ohne die Beurteilung von Menge und Qualtiät des Urins zu behindern, der günstig zu fertigen und einfach in der Handhabung ist.

Überraschend wurde nun gefunden, dass diese Aufgabe durch eine flexible, undurchsichtige Folie gelöst werden kann, welche dieselben Abmessungen wie der Urinbeutel hat und nur am oberen Rand des Beutels befestigt ist, so dass die Folie einen frei fallenden Vorhang bildet.

Die Erfindung löst daher die obigen Aufgaben durch einen Hygienbeutel mit einem Sichtschutz, der aus einer flexiblen, undurchsichtigen Folie mit im wesentlichen denselben Abmessungen wie der Flüssigkeitssammelbehälter des Hygienebeutels besteht, wobei die Folie nur am oberen Rand des Flüssigkeitssammelbehälters befestigt ist.

Die Folie kann, da sie flexibel und nur am oberen Rand des Behälters befestigt ist, zur Beurteilung der Menge und/oder Qualität der gesammelten Körperflüssigkeit einfach angehoben werden, wobei die gesamte durchsichtige Vorderseite des Behälters freigelegt wird. Die Folie fällt beim Loslassen von allein wieder herunter.

Auch bei der Entleerung stört die Folie nicht. Sie ragt nicht oder nur sehr wenig über den unteren Rand des Beutels. Sie hat keinen unteren Abschluss, der zum Entleeren erst entfernt werden müsste, das Pflegepersonal kann den Abflusshahn direkt erreichen. Wird eine Folie aus Kunststoff verwendet, wäre sie im Bedarfsfall auch einfach abwischbar. Bei den bevorzugten Befestigungsvarianten kann die Folie auch zum Abspülen abgenommen oder schlicht ausgewechselt werden. Ausnehmung oder einen durchsichtigen Bereich auf. Dadurch wird die Beurteilung der Füllmenge noch weiter erleichtert.

Die Folie wird vorzugsweise aus Kunststoff, insbesondere aus dem gleichen Kunststoff wie der Beutel, gefertigt. Geeignet sind beispielsweise PVC, Polyolefine wie Polyethylen oder Polypropylen, usw. Die Folie kann aber auch aus anderen Materialien, z.B. Vlies, Gewebe und ähnlichem bestehen.

Wichtig ist, dass es sich um ein undurchsichtiges Material handelt, damit ein ausreichender Sichtschutz erreicht wird. Außerdem darf die Folie nicht zu steif sein, damit sie an der Oberfläche des Beutels anliegt.

Es versteht sich, dass für die Folienstärke ein Kompromiss aus Kosten (möglichst dünne Folien) und Stabilität bei der Nutzung (ausreichende Dicke für die Befestigung und zur Vermeidung von Beschädigungen) gewählt wird.

Die Befestigung erfolgt am oberen Rand des Hygienebeutels bzw. des Flüssigkeitssammelbehälters. Verschiedene Möglichkeiten hierfür sind dem Fachmann bekannt. Beispielsweise können Befestigungselemente wie Klebebänder, Klipse, Klammern und ähnliches an der Folie ausgebildet sein. Es ist ebenso möglich, zweitieilige Befestiger zu verwenden, bei denen Verbindungsmittel wie Druccknöpfe, Haken und Ösen, Klettverschlüsse, Knöpfe etc. an der Folie und an dem Beutel angebracht sind. Außerdem kann die Folie bei der Herstellung des Beutels direkt mit dem oberen Rand des Behälters verschweißt werden oder an ihrem oberen Rand mit dem oberen Rand des Behälters verklebt werden.

Jede der Möglichkeiten hat Vor- und Nachteile. Die Verschweißung bzw. Verklebung ist sehr haltbar. Es fallen keine Kosten für Befestigungsmittel bzw. nur die im Vergleich geringen Kosten für den Klebstoff an. Dafür müssen aber alle Beutel mit der Folie versehen werden, unabhägig davon, ob der Benutzer einen Sichtschutz benötigt/wünscht oder nicht.

Auch zweiteilige Verbindungsmittel sind sehr sicher, verursachen aber vergleichsweise hohe Kosten und die Verbindungsmittel am Beutel müssen ebenfalls immer angebracht werden, egal ob vom Benutzer ein Sichtschutz angebracht wird oder nicht. Dafür bieten die Verbindungsmittel die Möglichkeit, verschiedene Designs der Folie anzubieten, die Kosten für die Folie werden vermieden, wenn kein Sichtschutz verwendet wird. Die Folie kann wiederverwendet werden.

Befestiger nur an der Folie sind in Bezug auf die Kosten der Beutel am günstigsten und erlauben es, den Sichtschutz für sämtliche am Markt befindliche Hygienebeutel anzubieten. Auch sie erlauben eine Auswahl des Designs und eine Wiederverwendung.

Besonders bevorzugt sind Klipverbindungen und Folien mit einem Umschlag zum Aufstecken auf den oberen Behälterrand. Weiterhin sind Klettverschlüsse bevorzugt, wobei vorzugsweise die Folie die Hakenkomponente aufweist und die Schlaufenkomponente am Beutel angebracht ist, z.B. aufgeklebt ist.

Wie bereits erwähnt, muss die Folie in ihren Abmessungen an den jeweiligen Hygienebeutel angepasst werden. Es bietet sich bei Folien an, diese mit demselben Werkzeug wie die Beutelteile auszustanzen.

Natürlich können die Folien auch zum Zuschneiden vorgesehen werden, d.h. der Benutzer passt die Folie individuell an, was bei Folien aus Kunststoff, Vlies etc. mit einer Haushaltsschere problemlos möglich ist. Zu diesem Zweck können auch innen auf der Folie die Konturen der gängigsten Beutel aufgedruckt werden.

Es ist von Vorteil, dass bei den bevorzugten Befestigungsvarianten die Produktion der Hygienebeutel an sich nicht verändert werden muss. Die Folie kann zusammen mit dem Beutel verpackt werden. Sollen verschiedene Designs angeboten werden, ist es zweckmäßig, die Folien als Sortimente auszuliefern, aus denen Benutzer die Folie mit dem am besten gefallenden Desgin aussuchen können. Der Sichtschutz wird am häufigsten für Urinbeutel Anwendung finden. Er eignet sich aber genauso für Hygienebeutel, die zum Auffangen und Sammeln anderer Körperausscheidungen, wie beispielsweise Wundsekret, bestimmt sind.

Die Erfindung soll anhand der beigefügten Figuren näher erläutert werden, ohne jedoch auf die speziell beschriebenen Ausführungsformen beschränkt zu sein. Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

Es zeigen:
- Figur 1: einen Urinbeutel mit erfindungsgemäßem Sichtschutz
- Figur 2: den Urinbeutel von Figur 1 ohne Sichtschutz.

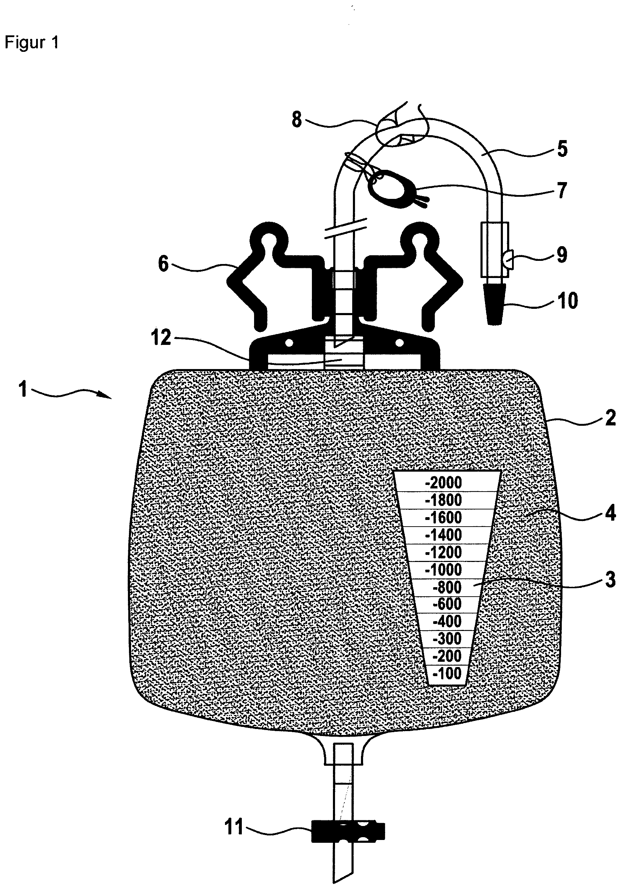

In Figur 1 ist ein Urinbeutel 1 gezeigt, wie er vom Grundaufbau her im Stand der Technik bekannt ist. Der Beutel 1 umfasst den Flüssigkeitssammmelbehälter 2, der von Folienstücken gebildet wird, die miteinander verschweißt sind. Auf dem Behälter 2 ist eine Skalierung 3 aufgedruckt, mit deren Hilfe der Füllstand beurteilt werden kann. Die Vorderseite des Behälters 2 ist ansonsten mit dem erfindungsgemäßen Sichtschutz in Form der Folie 4 abgedeckt. Oben sieht man den Schlauch 5, welcher den Anschluss zum Katheter (nicht gezeigt) herstellt. Außerdem verfügt der Beutel 1 über eine Aufhängung 6, eine Schlauchfixierklemme 7, eine Ritsch-Ratsch-Klemme 8, eine Probenentnahmestelle 9 und eine Schutzkappe 10. Unten befindet sich der Ablasshahn 11, über den der Beutel 1, genauer der Behälter 2, entleert werden kann.

Es ist gut zu erkennen, dass die Folie 4 eine Ausnehmung hat, durch welche die Skalierung 3 sichtbar ist. Die Form der Folie 4 entspricht der Außenkontur des Behälters 2, so dass dieser, bis auf die Saklierung 3, vollständig abgedeckt ist. Der Ablasshahn 11 ist gut zugänglich, so dass eine Entleerung problemlos erfolgen kann.

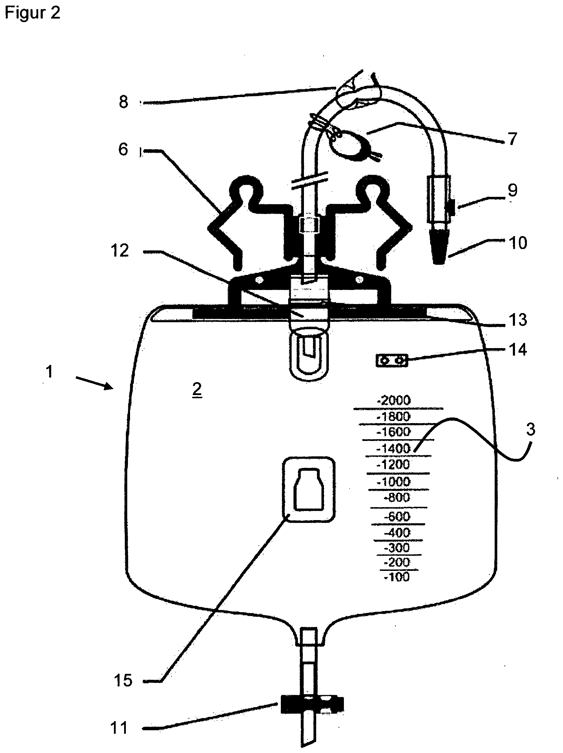

In Figur 2 ist der Beutel 1 von Figur 1 ohne Folie 4 gezeigt. Man erkennt neben den schon beschriebene Teilen, die nicht nochmals erläutert werden, die Tropfkammer 12, ein Antirefluxventil 13 und eine Belüftung 14. Da die Folie 4 lose an dem Behälter 2 anliegt, behindert der erfindungsgemäße Sichtschutz die Belüftung wenig oder gar nicht, obwohl die Belüftung 14 von der Folie 4 verdeckt ist. Der Halter 15 für den Ablasshahn 11 kann im Prinzip ebenfalls weiter benutzt werden, ohne dass ein relevanter Zusatzaufwand für die Entleerung entsteht. Da die Folie 4 nur am oberen Rand des Beutels 1 befestigt ist, kann sie zum Herausnehmen des Hahnes 11 aus der Halterung 15 einfach hochgehoben werden. Auch zur Beurteilung der Qualität das Urins wird die Folie 4 angehoben. Beim Loslassen fällt sie aufgrund der Schwerkraft von allein in die gewünschte Lage zurück, in welcher der Behälter 2 abgedeckt ist.

### Bezugszeichenliste

- 1: Urinbeutel
- 2: Flüssigkeitssammelbehälter
- 3: Skalierung
- 4: Folie
- 5: Schlauch
- 6: Aufhängung
- 7: Schlauchfixierklemme
- 8: Ritsch-Ratsch-Klemme
- 9: Probenentnahmestelle
- 10: Schutzkappe
- 11: Ablasshahn
- 12: Tropfkammer
- 13: Antirefluxventil
- 14: Belüftung
- 15: Halter für Ablasshahn

## Patentansprüche

1. Hygienebeutel mit einem Sichtschutz, der aus einer flexiblen, undurchsichtigen Folie (4) mit im wesentlichen denselben Abmessungen wie ein Flüssigkeitssammelbehälter (2) des Hygienebeutels (1) besteht, **dadurch gekennzeichnet, dass** die Folie (4) nur am oberen Rand des Behälters (2) befestigt ist.

2. Hygienebeutel gemäß Anspruch 1, **dadurch gekennzeichnet, dass** die Folie (4) aus Kunststoff, insbesondere aus dem gleichen Kunststoff wie der Behälter (2), gefertigt ist.

3. Hygienebeutel gemäß Anspruch 1, **dadurch gekennzeichnet, dass** die Folie (4) aus Vlies oder Gewebe ist.

4. Hygienebeutel gemäß mindestens einem der Ansprüche 1 bis 3, **dadurch gekennzeichnet, dass** Befestigungselemente wie Klebebänder, Klipse, Klammern oder Umschläge zur Befestigung der Folie (4) an dem Behälter (2) ausgebildet sind.

5. Hygienebeutel gemäß mindestens einem der Ansprüche 1 bis 3, **dadurch gekennzeichnet, dass** Verbindungsmittel wie Druckknöpfe, Haken und Ösen, Klettverschlüsse oder Knöpfe an der Folie (4) und an dem Behälter (2) angebracht sind.

6. Hygienebeutel gemäß mindestens einem der Ansprüche 1 bis 3, **dadurch gekennzeichnet, dass** die Folie (4) mit dem oberen Rand des Behälters (2) verschweißt oder verklebt ist.

7. Hygienebeutel gemäß mindestens einem der Ansprüche 1 bis 6, **dadurch gekennzeichnet, dass** der Beutel (1) eine Skalierung (3) aufweist und die Folie (4) über der Skalierung (3) eine Ausnehmung oder einen durchsichtigen Bereich aufweist.

## Claims

1. Sanitary bag with a privacy screen consisting of a flexible, opaque film (4) of substantially the same dimensions as a liquid collecting container (2) of the sanitary bag (1), **characterized in that**
the film (4) is fixed only to the upper edge of the container (2).

2. Sanitary bag according to claim 1, **characterized in that** the film (4) is made of plastic, in particular of the same plastic as the container (2).

3. Sanitary bag according to claim 1, **characterized in that** the film (4) is made of a non-woven or fabric.

4. Sanitary bag according to at least one of claims 1 to 3, **characterized in that** fastening elements such as adhesive tapes, clips, clamps or flaps are formed on the container (2) for fastening the film (4).

5. Sanitary bag according to at least one of claims 1 to 3, **characterized in that** connecting means such as press buttons, hooks and eyes, hook and loop fasteners or buttons are mounted on the film (4) and on the container (2).

6. Sanitary bag according to at least one of claims 1 to 3, **characterized in that** the film (4) is welded or glued to the upper edge of the container (2).

7. Sanitary bag according to at least one of claims 1 to 6, **characterized in that** the bag (1) has a scale (3) and the film (4) has an opening or a transparent area above the scale (3).

## Revendications

1. Sachet hygiénique avec protection contre la vue constitué d'une feuille souple opaque (4) présentant des dimensions essentiellement semblables à un collecteur de liquide (2) du sachet hygiénique (1),
**caractérisé en ce que**
la feuille (4) est fixée uniquement au bord supérieur du collecteur (2).

2. Sachet hygiénique selon la revendication 1, **caractérisé en ce que** la feuille (4) est fabriquée en matière synthétique, en particulier en la même matière synthétique que le collecteur (2).

3. Sachet hygiénique selon la revendication 1, **caractérisé en ce que** la feuille (4) est en non-tissé ou en tissu.

4. Sachet hygiénique selon au moins l'une des revendications 1 à 3, **caractérisé en ce que** des éléments de fixation comme des bandes collantes, des agrafes, des pinces ou des enveloppes sont réalisés pour la fixation de la feuille (4) au collecteur (2).

5. Sachet hygiénique selon au moins l'une des revendications 1 à 3, **caractérisé en ce qu'**on ajoute des moyens de raccord comme des boutons pression, des crochets et des oeillets, des fermetures Velcro ou des boutons à la feuille (4) et sur le collecteur (2).

6. Sachet hygiénique selon au moins l'une des revendications 1 à 3, **caractérisé en ce que** la feuille (4) est soudée ou collée avec le bord supérieur du collecteur (2).

7. Sachet hygiénique selon au moins l'une des revendications 1 à 6, **caractérisé en ce que** le sachet (1) présente une graduation (3) et la feuille (4) présente au-dessus de la graduation (3) un vide ou une zone transparente.
